# EUROPEAN PATENT APPLICATION

(11) **EP 4 781 828 A1**
(43) Date of publication of application: **29.07.2026**
(21) Application number: 25305100.7
(22) Date of filing: 27.01.2025
(51) Int. Cl.: A01N 63/23, A01N 63/50, C07K 14/32, C07K 14/325, C12N 15/82

(54) **NON-SPORULATING, NON-LYSING BACILLUS THURINGIENSIS STRAIN AND ITS USE AS BIOPESTICIDE**

(71) Applicant: Institut National de la Recherche pour l'Agriculture, l'Alimentation et l'Environnement, 75007 Paris (FR); Institut National des Sciences et Industries du Vivant et de l'Environnement, 91120 Palaiseau (FR); Université Paris-Saclay, 91190 Gif-sur-Yvette (FR)
(72) Inventor: VERPLAETSE, Emilie, 91400 Saclay (FR); LERECLUS, Didier, 28260 Oulins (FR); SLAMTI, Leyla, 75015 Paris (FR)
(74) Representative: Gevers & Orès

(57) **Abstract**

The present invention relates to a genetically modified *Bacillus thuringiensis* strains having an improved spectrum of action against insect crop pests and its use as a biopesticide. More specifically, these strains are such that they do not form viable and resistant spores and do not express endogenous N-acetylmuramoyl-L-alanine amidases regulated by VipR.

## Description

The present invention relates to a genetically modified *Bacillus thuringiensis* strains having an improved spectrum of action against insect crop pests and its use as a biopesticide. More specifically, these strains are such that they do not form viable and resistant spores and do not express endogenous N-acetylmuramoyl-L-alanine amidases regulated by VipR. As a consequence, they do not lysate and the insecticidal toxins produced by the strain are retained in the unsporulated bacterial cell.

*Bacillus thuringiensis* (Bt) is a Gram-positive spore-forming bacterium that produces large quantities of insecticidal proteins (Cry and Cyt toxins). These toxins accumulate in the bacteria, forming crystalline inclusions representing up to 25% of the bacterium's dry weight. These inclusions are released by cell lysis at the end of the sporulation process (Agaisse and Lereclus, 1995). In addition to these toxins, some Bt strains produce insecticidal toxins that do not form crystalline inclusions and are partially exported in the culture medium, such as Vip3 and Cryll (Estruch *et al.,* 1996; Wang *et al.,* 2021).

Cry1l toxins belong to the Cry1 family of 3-domain toxins, due to their sequence similarity and structural characteristics. However, these toxins are not part of the typical Bt crystals and are produced as soon as Bt enters the stationary phase of growth and are found in the culture medium (Kostichka *et al.,* 1996). Cryll toxins have the particularity of exhibiting dual insecticidal activity, on Coleoptera and Lepidoptera larvae (Tailor *et al.,* 1992), and they recognize different receptors from other Cry1 toxins in the gut of sensitive insects (Guo *et al.,* 2011, Ruiz de Escudero *et al.*, 2006). No cross-resistance was observed with Cry1A and Cry1F toxins, supporting the value of using these toxins in gene stacking strategies in new insecticide products (Carrière *et al.,* 2016).

Like 3-domain toxins such as Cry1 and Cry2, Vip3A toxins are active against lepidopteran larvae, but do not bind to the same receptors on insect larval gut epithelial cells as Cry toxins (de Maagd *et al.,* 2003; Gouffon *et al.,* 2011). As a result, Vip3A toxins are an interesting complement to Cry toxins in the control of lepidopteran insects, and are used in the construction of transgenic plants by stacking insecticidal toxin genes. They enable to broaden the insecticidal activity spectrum of the transgenic plants, notably by targeting major crop pests (Noctuidae insects such as *Spodoptera frugiperda*)*.* They also reduce the risk of resistant insects appearing, as they do not recognize the same receptors as Cry toxins. Very low concentrations of Vip3A toxin are active on sensitive Lepidoptera larvae: 4 ng/cm² of food leads to paralysis of the digestive tract, and 40 ng/cm² of food leads to 50% mortality.

The main biopesticide (the Bt kurstaki HD1^{®} strain) marketed today to control insect crop pests produces the Cry1A, Cry2A, Vip3A and Cryll toxins. Whereas the Cry1A and Cry2A toxins form a protein crystal, the Vip3A and Cryll toxins do not form crystals and are mainly released into the bacterial growth medium. At present, Bt-based biopesticides are composed of spores and free crystals. As a result, only traces of Vip3A and Cry1l proteins are found in biopesticide formulations. It thus remains a need to provide improved Bt-based biopesticides comprising a larger diversity of toxins and extending the strain's spectrum of action against insect crop pests.

Inventors have now identified that the control of the expression of a specific family of amidases whose transcription is regulated by the VipR regulator can prevent the lysis of Bt occurring early in the stationary phase of growth and then the release of toxins of interest in the culture medium; the invention therefore makes it possible to retain a greater proportion, if not all, of the toxins inside the cell, and to increase the number of bacterial cells containing toxins at the end of culture. Advantageously, the toxins are thus protected from degradation and fully active against target insects. As Cryll has dual activity against lepidopteran and coleopteran larvae, the strain's spectrum of action is thus broadened.

More specifically, the present invention relates to a genetically modified non-sporulating and non-lysing *Bacillus thuringiensis* strain comprising one or more *ami* genes each coding for an Ami protein (i) having at least 70%, preferably at least 75%, 80%, 85%, 90%, 95%, 98% or 100% identity with the sequence SEQ ID N°1 and (ii) whose transcription is regulated by the VipR regulator,
wherein the expression or activity of said protein is abolished.

The *Bacillus thuringiensis* strain is preferably chosen among the following serovars: kurstaki such as HD1, HD73, SA-11, SA-12, PB54, EG2348 or ABTS-351 strains, aizawai such as ABTS-1857 or GC91 or 7.29 strains, galleriae such as HD-29, thuringiensis such as ATCC10792 strain, alesti such as BGSC 4C1 strain, chinensis such as CT-43 strain.

Preferably, the strain is Bt aizawai ABTS-1857 or GC91 or 7.29 or Bt kurstaki HD1, HD73, SA-11, SA-12, PB54, EG2348 or ABTS-351.

In order to suppress the sporulation activity of a strain of the genus *Bacillus,* it is possible to inactivate any gene essential for sporulation such as the genes involved in the expression of the Spo0A transcriptional regulator responsible for the initiation of sporulation or in the expression of the SigE, SigF, SigG, SigH and SigK sporulation sigma factors or to abolish the activity of the proteins encoded by these genes; preferably, the inactivated gene is *Spo0A* or *sigE* or *sigK*; more preferably, the inactivated gene is *Spo0A.*

In a general way, genetic modification of bacterial strain to abolish the expression of a gene or the activity of a protein can be achieved by the following methods.

The inactivation of a gene can be obtained by interruption or modification of the coding sequence, by deletion of all or part of the gene, or by removal or modification of the promoter elements. Such deletion can be obtained by homologous recombination between the adjacent regions located upstream and downstream of the gene, using plasmids whose replication is thermosensitive, for example the plasmids pRN5101 (Lereclus et al., Biotechnology (NY) 10: 418-421,1992) or pMAD (Arnaud et al., Appl Environ Microbiol 70: 6887-6891,2004), and using the protocols described in these articles or non-replicative plasmids like pUC18 or conjugative plasmids such as pAT28 (Trieu-Cuot et al. Nucleic Acids Res. 1990 Jul 25;18(14):4296. doi: 10.1093/nar/18.14.4296).

This abolishment of the expression and/or activity of a protein can also be obtained by mutagenesis, by deletion, insertion and/or substitution of one or more nucleotides of the gene encoding this protein. This mutagenesis can then occur at the level of the coding sequence or the sequences regulating the expression of the gene, in particular at the level of the promoter, leading to a loss of transcription or translation of the protein. For example, the introduction of one or more point mutations within the coding sequence of a gene or its expression regulation sequences can induce, depending on the nature of the mutation, a shift of the reading frame and/or the introduction of a stop codon in the sequence and/or the lack of the transcription or translation of the genes encoding the protein and/or a substitution of one of the amino acids of the active site of the protein and lead to the expression of an inactive protein, or an protein with a decreased activity.

Mutagenesis can be implemented by inducing random mutations, for example using physical agents, such as radiation or chemical agents such as EMS (Ethyl Methane Sulfonate) or using PCR or in a targeted manner by transfection, transduction, natural transformation or electroporation (Bron *et al.,* 2019). Alternatively, mutagenesis can be implemented by methods using nucleases (TALEN, CRISPR/Cas9, Wei *et al.*, 2013).

Methods for deleting, inserting and/or substituting a given genetic sequence in bacteria are well known to the skilled person.

The Bt according to the invention can also be obtained by modification of the promoter sequence of the gene according to for example one of the methods used to substitute a nucleotide sequence cited above, or by an RNA interference (RNAi) technique, by expression of an antisense RNA or by aptamers.

In the present invention, such methods can in particular be applied to obtain a non-sporulating strain and to abolish the expression or activity of the Ami proteins.

Deletion of the *Spo0A* gene (designated Δ*spo0A*) has a very early effect, as soon as the bacteria enter into the stationary phase, notably preventing the bacteria from engaging in the sporulation process (Lereclus et al., Biotechnology (NY) 13: 67-71, 1995). Deletion of the *sigE* gene (designated *ΔsigE*) or of the *sigK* gene (*ΔsigK*) has a later effect, blocking the progression of the sporulation process (Bravo et al., Mol Gen Genet 250: 734-741, 1996). In each case, the bacteria die and contain mostly the toxins of interest.

Accordingly, the Bt strain of the invention is such that the sporulation gene chosen from *spo0A, sigE, sigF, sigG, sigH* and *sigK,* preferably from *spo0A, sigE* and *sigK,* more preferably *spo0A,* is inactivated by interruption or modification of its coding sequence or by deletion of all or part of the gene or the activity of at least one protein encoded by these genes is abolished.

The loss of activity of Spo0A, SigE, SigF, SigG, SigH or SigK can be achieved by the deletion of a gene coding for a protein identified as involved in post-translational modifications leading to the activation of these transcriptional regulators. For example, Spo0A can be inactivated by the deletion of the gene coding for SpoOF or Spo0B, two proteins involved in the phosphorylation cascade leading to the activation of Spo0A (Burbulys, Cell, 1991; Malvar, JB, 1994); SigE inactivation can be realized by the deletion of the gene coding for SpollGA, a protein required for proteolytic cleavage of pro-SigE (Peters and Haldenwang, JB, 1994 10.1128/jb.176.24.7763-7766.1994 ); SigK can be inactivated by the deletion of *spolVFB,* a gene coding for a metalloprotease involved in the conversion of the pro-SigK into its active form (Prince, JB https://doi.org/10.1128/jb.187.3.961-971.2005)

The Ami proteins are proteins having at least 70%, preferably at least 75%, 80%, 85%, 90%, 95%, 98% or 100% identity with the sequence SEQ ID N°1 as follows and encoded by an *ami* gene:

SEQ. ID. N°1: 
MTVFNX1KEINX2X3PFESNIIPKGNRNRPAYX4MNPX5YITIHTTANQNAGX6DARAHARYVNNGGGSX4X7VS
WHFTVDDX8RIVQHLPLNENGWHAGDGNGAGNRSSIGIEICENRDGNFEKALTHAAGLVKFLMAHLGLKIGN
VVSHQHWSGKNCPRPIFNRPGGFEGFKRMIEDSX9NDMVVENHNKDAGTQGLGIAYMEGVNINLRRGPSTSS
DVIRKLNKPESYIVWEERX10GWLX11LGNSWVKYDX12SYIYFARRX13TSNX14GX15LVVVDX6NELWVYGSAX11
WNX11X15X14KTVKKGEAFTIX16DELQX14NGSNMYKCKYFYITX6NSQFVYVK
 Where
X₁ is T or V
X₂ is K or N
X₃ is I or T
X₄ is S or T
X₅ is G or R
X₆ is A or T
X₇ is G or S
X₈ is H or N
X₉ is L or P
X₁₀ is D or E
X₁₁ is D or N
X₁₂ is P or S
X₁₃ is K or Q
X₁₄ is I or V
X₁₅ is K or R
X₁₆ is L or Q

These proteins are endogenous proteins showing structural similarity with N-acetylmuramoyl-L-alanine amidases. These proteins will be designated as Ami in the subsequent parts of this patent. N-acetylmuramoyl-L-alanine amidases are enzymes of the peptidoglycan hydrolase family that hydrolyze the bonds between N-acetylmuramoyl and L-amino acids (preferentially D-lactyl-L-alanine) residues present in the wall of certain bacteria. These enzymes are present throughout bacterial genomes and are involved in a large number of biological processes such as wall synthesis, cell division, and genetic transformation (Vermassen A et al. 2019).

Examples of such Ami proteins are proteins of SEQ. ID. N°3, 4, 5, 6, 7, 8, 9, 10, 11 and 12.

The transcription of the genes encoding this specific family of N-acetylmuramoyl-L-alanine amidases is regulated by the VipR regulator. This regulation involves a VipR-box comprising a sequence: 5' TTC-N-N-N-N-AT-N-G-N-N-GAA-N-N-TAT-N-T-N-T-N-CTTT 3' (SEQ ID N°2), wherein N is A or T or C or G, and wherein said sequence of SEQ ID N°2 is positioned between 15 and 21 nucleotides upstream of the Sigma A-specific -10 box preceding the start codon of the *ami* gene and more preferably 17 nucleotides. The Sigma A-specific -10 box has the following sequence: TNNNNT with N is C or A or G or T, preferably TATAAT or TATACT or TATGCT or TATCGT or TATCTT or TATATT or TATTAT or TATAGT or TATGAT and more preferably TATAAT or TATACT.

In case the Bt strain according to the invention produces one or several Ami proteins, expression and/or activity of each of these Ami proteins has to be abolished to prevent the lysis of the bacterial cells. For example, such abolishment can be obtained by any of the methods described above. According to the invention, the transcription of the *ami* genes is regulated by the VipR regulator. VipR is an autoregulated transcriptional regulator that activates the expression of the *vip3A* gene at the onset of stationary growth phase, due to the presence of a VipR-box 17-bp upstream from the promoter. VipR is also involved in the control of several insecticidal toxin genes present in the strain's plasmids and preceded by a canonical VipR-box: *cry1la, cry2Ab,* the operon containing *cry2Aa* and 3 operons containing an *ami* gene and a *cry1A* gene (Chen, Verplaetse *et al.*, 2022, see example), making this regulator a major player in the insecticidal activity of the lepidopteran-active Bt strains.

The VipR regulator can be naturally present in the Bt strain according to the invention.

In case the Bt strain does not harbor the VipR regulator gene, the production of this regulator can be obtained by cloning the coding sequence and its promoter on a plasmid or in the chromosome of the Bt strain. Alternatively, the expression of *vipR* can be driven by another promoter active in a sporulation-deficient genetic context, for example, the promoter of *plcA, calY, oppA* or *nprA* genes. The insertion in the chromosome can be obtained by homologous recombination between the adjacent regions located upstream and downstream of the defined insertion point, using plasmids whose replication is thermosensitive, for example the plasmids pRN5101 (Lereclus et al., Biotechnology (NY) 10: 418-421,1992) or pMAD (Arnaud et al., Appl Environ Microbiol 70: 6887-6891,2004), and using the protocols described in these articles or non-replicative plasmids like pUC18 or conjugative plasmids such as pAT28 (Trieu-Cuot et al., NAR 2001). The cloning of the *vipR* gene and the selected promoter in a plasmid can be achieved by the Gibson cloning strategy, Golden Gate assembly, or by the classical method of production of cohesive ends in DNA fragments using restriction enzymes and the ligation of DNA fragments. Any plasmids that allows replication in Bt can be used, for example, pHT304 or derivatives such as pHT315 and pHT370 (Arantes and Lereclus, 1991 10.1016/0378-1119(91)90495-W) or pHT1618 (Lereclus D, Arantes O: Mol Microbiol. 1992, 6 (1): 35-46. 10.1111/j.1365-2958.1992.tb00835.x ).

The present invention also relates to a method for preparing a genetically modified non-sporulating and non-lysing *Bacillus thuringiensis* strain comprising the step of mutagenesis such that each gene encoding a proteins having at least 70%, preferably at least 75%, 80%, 85%, 90%, 95%, 98% or 100% identity with the sequence SEQ ID N°1 or its promoter is absent or present in a truncated form for abolishing in said bacterium the expression and/or activity of said proteins.

Methods of mutagenesis are as described above.

As detailed in the experimental part, Inventors have shown that, in VipR-containing strains, the deletion of a sporulation gene alone results in the production of non-sporulating bacteria producing a significant quantity of Cry toxins, and the additional deletion of VipR-regulated amidase genes, enables insecticidal toxins normally released into the growth medium, such as Vip3A and Cry1I, to be retained in the cell, but also the Cry1A and Cry2A crystal proteins that are produced at the onset of stationary phase, upon VipR activation of their expression.

These modifications enable the following improvements in Bt-based biopesticides that:
1- Enhance the insecticidal properties of these strains by combining Vip3 and Cryll proteins and Cry1 and Cry2 protein crystals;
2- Increase toxin persistence by encapsulation in the bacterial cell;
3- Avoid dissemination of bacterial spores in the environment; and
4- Reduce the probability of possible resistance of insects to Cry toxins and bypass the existing resistance.

Accordingly, the present invention further relates to the use of a genetically modified non-sporulating and non-lysing *Bacillus thuringiensis* strain according to the present invention as a biopesticide (also designated Bt biopesticide).

"Biopesticide" in the present invention refers to pesticides derived from natural materials, such as bacteria, for example. This general term "biopesticide" includes the term "biocide" which is more used in a context of controlling vectors, such as vectors that transmit pathogens responsible for mammalian diseases.

In a particular embodiment, the strain of *Bacillus thuringiensis* according to the present invention is used for protecting plant cultures against insect pests.

The Bt biopesticide of the invention can be used for controlling crop pests, specifically lepidopteran insects such as those belonging to the families *Tortricidae, Noctuidoe* and *Pyralidae* which damage crops like cotton, cabbage, corn, soybean, grapevine and any other extensive or gardener cultures and Coleoptera insects such as *Leptinotarsa decemlineata* which damage crops like potatoes, eggplants, or tomatoes, or *Anthonomus grandis,* a major threat for cotton plants.

Preferably, the toxins produced by the recombinant strain of the invention for protecting cultures are: Cry1, Cry2 and Vip3 toxins and preferentially Cry1A, Cry1C, Cry1D, Cry1I, Cry2A and Vip3A. The uses as biopesticide encompassed by the present invention are not method for treatment of the human or animal body by therapy.

### Figures

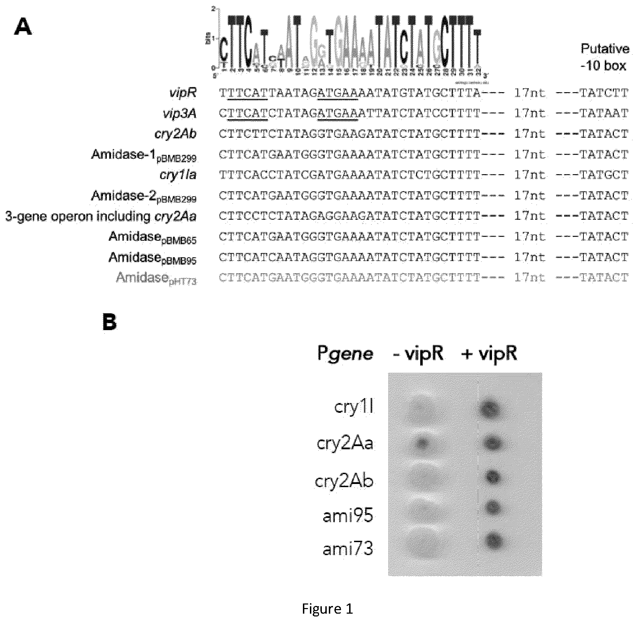

**Figure 1****: A.** Alignment of the conserved VipR-box sequences present on the pBMB299 and pHT73 plasmids. The name of the genes controlled by VipR is indicated on the left. The distance between the putative -10 box and the last nucleotide of the conserved motif is indicated. A consensus is shown on top as a sequence logo in which the height of the letters in bits is proportional to their frequency.
   **B.** The strains HD73- harboring the plasmids pPgene-lacZ and the pHT-Pxyl-vipR (column « + vipR ») or the pHT-Pxyl (column « - vipR ») were patched on LB medium supplemented with X-gal (50 µg/mL) and xylose 20 mM, and grown at 30°C for 24 h and at 20°C for another 24h. The name of the genes whose promoter is fused to *lacZ* is indicated on the left. The darker aspect of the colony obtained in the presence of *vipR* indicates production of a blue coloration, a proxy to detect the transcriptional activity of the promoter. Picture was taken after 48h of growth.

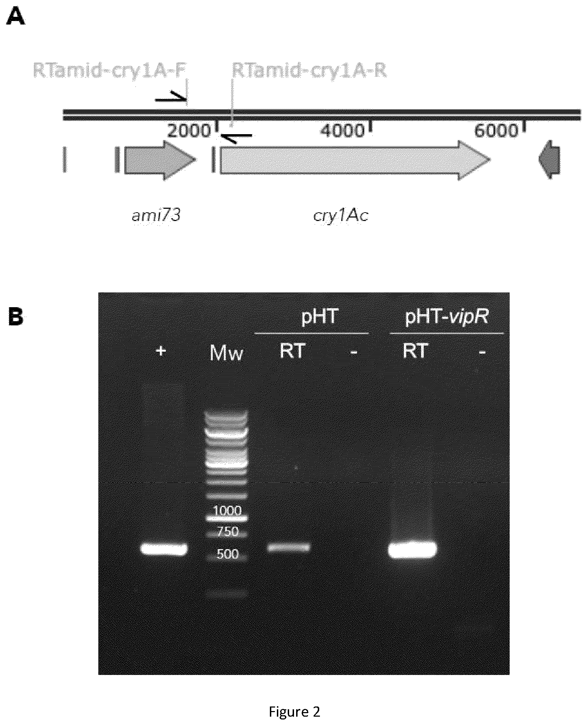

**Figure 2****: A.** Schematic of the *ami73-cry1Ac* locus present on the pHT73. The small arrows indicate the location of the primers used for the PCR.
   **B.** RT-PCR detection of the *ami73-cry1Ac* transcription unit in HD73. Agarose gel electrophoresis analysis of the production of a 600-bp PCR product obtained with the RTamid-cry1A-F et RTamid-cry1A-R primer pair and HD73 genomic DNA as the DNA template (lane +), or HD73 (pHT) and HD73 (pHT-vipR) RNA treated, or not, with the reverse transcriptase (lanes RT and -, respectively). RNAs were collected 2 hours after the onset of stationary phase from bacteria grown in LB medium at 30°C. Mw, DNA molecular weight ladder.

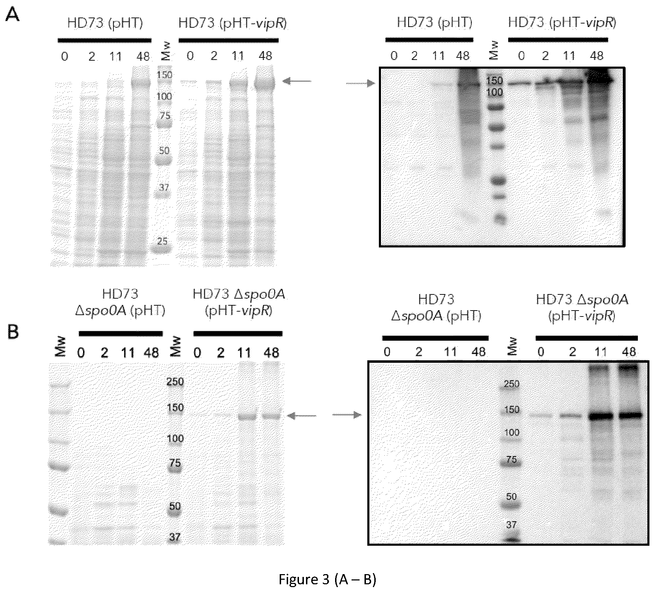

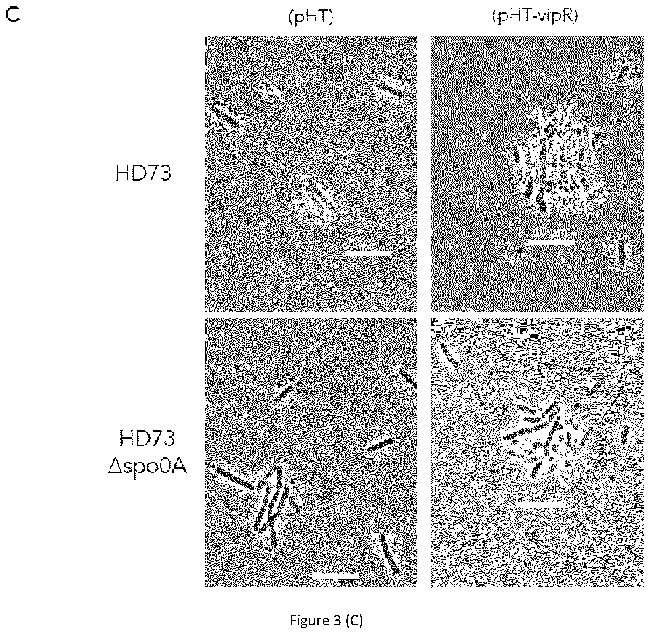

**Figure 3****: A.** Total protein extracts from the HD73 strain that carries the pHT-*vipR*, or the pHT. Samples were collected at the indicated time (in hours; "0" corresponding to the onset of stationary phase, *ie*. when bacterial growth starts to decline) and analyzed by SDS-PAGE (on the left) and Western blotting (on the right) using an antiserum raised against the Bt *aizawai* 7.29 crystals and able to detect Cry1A proteins. Arrows point to protein bands that correspond to Cry1Ac. Strains were grown at 30°C on LB medium and erythromycin (5 µg/mL).
   **B.** Total protein extracts from the HD73 Δ*spo0A* strain carrying the *vipR* gene (pHT-*vipR*), or the plasmid alone (pHT). Samples were collected and analyzed as described in A.
   **C.** Microscopy observations of the above-mentioned strains grown in LB medium at 30°C for 48h. Arrowheads point to typical Cry1Ac bipyramidal crystals.

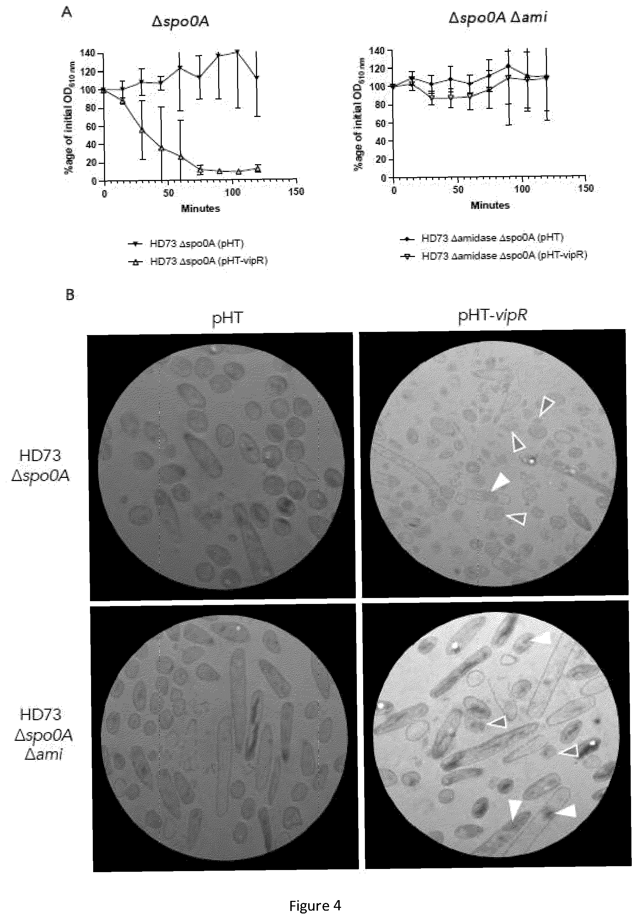

**Figure 4****:** Autolysis analysis of the HD73 Δ*spo0A* et HD73 Δ*spo0A* Δ*ami* strains carrying the pHT-*vipR* or the pHT.
   **A.** The autolysis rate (measured as the drop of the optical density of the bacteria suspension at 610 nm over time) of bacteria collected 2 h after the onset of stationary phase was determined in saline over 2 h at 30°C without shaking. The bacterial suspension was shaken before each optical density measurement. Data are presented as the mean ± SD (n = at least 4).
   **B.** Electronic transmission microscopy observations of the above-mentioned strains grown in LB medium at 30°C for 48 h. Grey arrowheads point towards typical Cry1Ac bipyramidal crystals that are not surrounded by a bacterial envelope. White arrowheads point to crystals located inside an intact bacterial envelope.

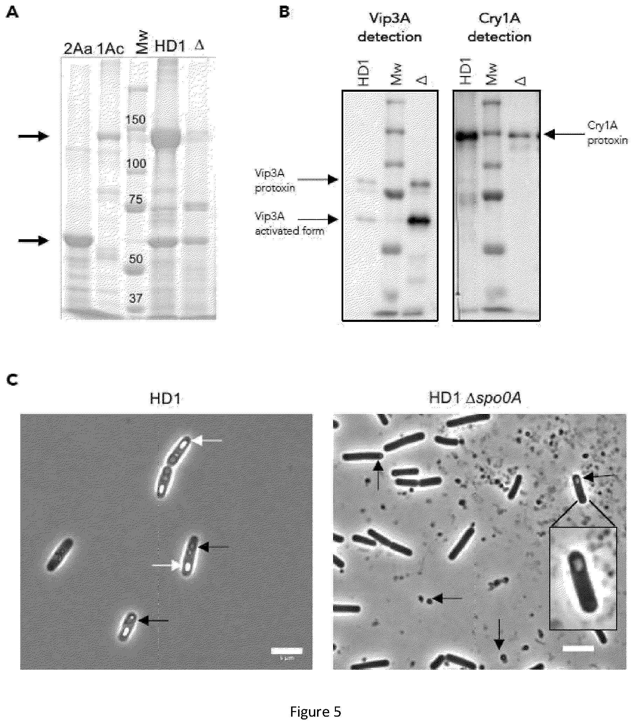

**Figure 5****:** Production of insecticidal proteins by a sporulation-deficient HD1 strain.
   **A.** SDS-PAGE analysis of 5 µg of a total protein extract of the HD1 (lane HD1) and HD1 *ΔspoOA* (Lane Δ) strains. Bacteria were grown on LB medium plates at 30°C for 2 days and followed by an incubation at room temperature for 6 days. Lane 1Ac, total protein extract of the HD73 strain that produces only Cry1Ac. Lane 2Aa, total protein extract of a recombinant *Bacillus thuringiensis* strain that produces only Cry2Aa. Arrows point to the 133-kDa Cry1Ac protein and the 70-kDa Cry2Aa protein. Mw, protein molecular weight marker.
   **B.** Western blotting using a monoclonal antibody raised against Vip3A (left panel) or an antiserum raised against the Bt aizawai 7.29 crystals and able to detect Cry1A proteins (right panel). Arrows point to protein bands that correspond to Vip3A (protoxin or the activated form) and Cry1A. Strains were grown as described in A. Respectively 0,15 µg and 1,21 µg of a total protein extract of the HD1 and HD1 Δ*spo0A* strains was loaded onto the gel and transferred onto PVDF membrane.
   **C.** Microscopy observations of the HD1 WT and Δ*spo0A* strains grown on LB plates at 30°C for 48 h. Black arrows points to crystalline inclusions, liberated or not. White arrows point to endospores. White bar corresponds to 5 µM.

### Examples

**The transcriptional regulator VipR controls the expression of operons comprising an *ami* gene and a *cry1A* gene in *Bt kurstaki* strains HD1 and HD73.**

The VipR transcriptional regulator of *Bacillus thuringiensis kurstaki* HD1 strain positively controls the expression of the *vip3A* gene located on the same plasmid as well as its own expression. A 32-bp conserved sequence, called the VipR-box, critical for gene expression was identified in the *vipR* and *vip3A* promoter and this sequence was also identified in the promoter of 4 other transcription units on the pBMB299 plasmid: upstream of *cry2Ab, cry1I,* a three-gene operon containing *cry2Aa* and in the promoter of a gene coding for a protein showing similarities with a N-acetylmuramoyl-L-alanine amidase (abbreviated as *ami299* coding for protein of SEQ. ID. N°6), all genes being located in a pathogenicity island. The presence of a VipR-box upstream from those genes suggested that these genes are regulated by VipR as well (Chen, Verplaetse *et al.,* 2022).

Analysis of the genome of strain HD1 also revealed the presence of this VipR-Box in the promoter region of 2 other genes coding for putative amidases present on pBMB65 and pBMB95 **(****Fig. 1A****).** These genes are named *omi65* (coding for protein of SEQ. ID. N°8) and *omi95* (coding for protein of SEQ. ID. N°7). To verify the control of expression of these target genes by the VipR regulator, the DNA fragments containing the VipR-box and located directly upstream of 2 *ami* genes, *cry1I, cry2Ab* and the operon containing *cry2Aa* were cloned upstream of the *lacZ* gene on plasmid pHT304.18Z and these constructs were introduced into a heterologous strain that does not naturally contain the *vipR* gene, strain HD73. A plasmid allowing the expression of the VipR regulator under the control of a xylose-inducible promoter, pHT16.18-Pxyl-vipR, and a control plasmid that does not contain the coding sequence of the regulator, pHT16.18-Pxyl, were then introduced into the strains containing the transcriptional fusions. The resulting strains were isolated on LB plates containing 50 µg/ml X-gal, and the plates were observed after 24 h incubation at 30°C and after a further 24 h incubation at 20°C. At 24 h, a blue staining was present in all strains expressing the *vipR* gene. At 48 h, a blue coloration was also observed in strain HD73⁻ (pPcry2Aa-*lacZ*, pHT16.18-P*_{xyl}*), in the absence of the VipR regulator **(****Fig. 1B****).** This shows that the promoters of the *cry1I, cry2Ab* and *omi95* target genes are only activated in strain HD73 in the presence of the VipR regulator. In contrast, the transcription of the *cry2Aa* operon is first activated by the VipR regulator before a sporulation-dependent factor, likely SigE, in turn activates its expression. This VipR-independent transcription could be due to the presence of the SigE-dependent promoter located downstream of the VipR-box in the promoter of the *cry2Aa* operon.

An analysis of the genome of strain HD73, which does not naturally contain the *vipR* gene, led to the identification of a VipR box on pHT73. This sequence is located upstream of a gene coding for a putative N-acetylmuramoyl L-alanine amidase. Using a plasmid transcriptional fusion between the promoter of this gene, designated *ami73* (coding for protein of SEQ. ID. N°3), and *lacZ,* it was confirmed that VipR was involved in controlling the expression of this protein **(****Fig. 1B****),** indicating that even if the strain does not naturally contain the regulator, transcription of the gene can be restored by introducing the regulator into the strain.

Interestingly, it has been found that these VipR-regulated *ami* genes from strains HD1 and HD73 are systematically located upstream of a *cry1A*-type gene and the genetic organisation of these two genes suggested that they could form a single transcription unit. In addition, a bioinformatics analysis did not reveal any sequence that could correspond to a transcription terminator between these 2 ORFs. RT-PCR assays confirmed that the *ami73* and *cry1Ac* genes from strain HD73 did indeed form a single transcription unit **(****Fig. 2****).** RNA samples from a strain HD73 containing a plasmid construct allowing expression of *vipR* under its own promoter, strain HD73 (pHT-vipR), and from a strain containing a control plasmid not carrying the *vipR* expression cassette, strain HD73 pHT304.18 (pHT), were collected 2h after entry into the stationary phase of growth and reverse transcribed into cDNA. PCR was performed on these cDNAs using primers, one of which was located at the end of the *ami73* coding sequence (coding for protein of SEQ ID N°3) and the other at the start of the *cry1Ac* coding sequence. An amplicon has been produced covering the intergenic sequence indicating that these 2 genes belong to the same transcription unit.

The conservation of genetic elements between strains HD1 and HD73 suggests that these genes also form a transcription unit in Bt strain HD1.

**A sporulation mutant produces Cry1A crystals in the presence of VipR as soon as it enters the stationary phase of growth.**

It has long been established that the expression of the *cry1A* and *cry2* genes is controlled by sigma factors active during sporulation, Sigma E and Sigma K (Adams et al., 1991; Bravo, et al. 1996; Deng et al., 2017). Indeed, binding sequences for these sporulation sigma factors are present upstream of the coding sequences. In particular, in the case of the *cry1A* genes, a SigE-box and a SigK-box are present in the intergenic region between the amidase gene and *cry1A* and it has been shown that in the model strain Bt407, which lacks a *vipR* gene, deletion of the gene encoding SigE abolishes the expression of *cry1Aa* (Bravo et al., 1996).

The fact that the *ami* gene and *cry1A* could be expressed from the same transcript early in the stationary phase, and in a sporulation-independent manner, suggested that the Cry1A toxin could be produced in a strain that does not sporulate but possesses the *vipR* gene. The pHT-vipR has therefore been introduced into the HD73 *Δspo0A* sporulation mutant. A control strain HD73 *Δspo0A* (pHT) containing only the expression plasmid not carrying the *vipR* gene was also constructed. Strains HD73 (pHT-vipR) and HD73 (pHT), and strains HD73 *Δspo0A* (pHT-vipR) and HD73 *Δspo0A* (pHT) were grown in LB medium and we collected samples at T0, T4h, T11h and T48h. SDS-PAGE gel analysis of these samples showed that Cry1Ac toxin from strain HD73 was produced as early as T0 in the presence of VipR only, and in both genetic backgrounds, confirming our hypothesis **(****Fig. 3A and 3B****).** The identity of the band migrating at around 130 kDa has been confirmed by Western blot using an antiserum recognising Cry1A-type toxins. Microscopic analysis of strains HD73 (pHT) and HD73 (pHT-vipR) showed the presence of larger crystals in the latter. Observations of strains HD73 *Δspo0A* (pHT) and HD73 *Δspo0A* (*pHT-vipR*) confirmed the presence of crystalline-like structures only in the VipR-expressing sporulation mutant **(****Fig. 3C****).**

However, these microscopic observations highlighted that some of the crystals produced by strain HD73 *Δspo0A* (*pHT-vipR*) were released following lysis of part of the cells, whereas strain HD73 *Δspo0A* (pHT) did not lyse **(****Fig. 3C****).** In order to determine when lysis occurred, microscopic observations of strains HD73 *Δspo0A* (pHT) and HD73 *Δspo0A* (*pHT-vipR*) were performed during bacterial growth in LB medium. These observations showed that part of the bacterial population expressing the VipR regulator displayed a lysis phenotype 2h and 4h after entry into the stationary phase of growth (**Fig. 3D**). This phenotype corresponds to cells broken in 2 at their center or at one end, with the cytoplasm escaping through the breach formed. The control strain HD73 *Δspo0A* (pHT) did not exhibit this lysis phenotype.

These observations and their timing are linked to the VipR-dependent co-expression of the *ami73* located upstream of *cry1Ac* and suggests that the enzyme encoded by the *ami73* gene could be the inducer of this phenomenon.

### Deletion of the ami73 gene co-expressed with cry1Ac and controlled by VipR in strain HD73 abolishes bacterial lysis

Some Bt strains, including HD1, produce toxins that are released into the extracellular environment, such as Vip3 or Cry1I, however, the biological mechanism involved in the export of these toxins has not been characterized to date.

To determine the function of these putative amidases, the only gene of this kind, *ami73,* controlled by VipR and present in strain HD73, has been studied. A mutant of this gene was constructed in strain HD73 *Δspo0A.* The VipR expression plasmid, pHT-vipR, was then introduced into strain HD73 *Δspo0A Δami73.*

In order to quantify the lysis phenotype induced by Ami73 expression in the presence of VipR, an autolysis experiment was performed. The evolution of the optical density of a bacterial suspension of strains HD73 *Δspo0A* and HD73 *Δspo0A Δami73* in the presence of the pHT-*vipR* plasmid, or the empty plasmid, was followed over time **(****Fig. 4B****).** The results show that the HD73 *Δspo0A* (pHT-*vipR*) strain lyses (optical density falls) whereas the *Δspo0A Δami73 (pHT-vipR)* mutant does not lyse (optical density remains stable) over time as is the case with the HD73 *Δspo0A Δami73* (pHT) strain, which does not contain *vipR.* This result shows that Ami-like proteins controlled by VipR are involved in bacterial cell lysis in the early stationary phase. The conservation of bacterial wall integrity in strain HD73 *Δspo0A Δami73 (pHT-vipR)* compared with strain *Δspo0A* (pHT-vipR) was also observed by electron microscopy **(****Fig. 4B****).** It is observed that the presence of VipR results in the production of numerous crystals, some of which are very large in strain HD73 *Δspo0A* **(****Fig.4B****,** top panel) but many of these crystals are released and few intact cells are present in the preparation. In contrast, in strain HD73 *Δspo0A Δami73,* the bacterial wall appears to be intact in a large proportion of cells in the presence of VipR. A few released crystals can be identified but most are contained within a bacterial envelope **(****Fig.4B****,** bottom panel).

**The Bt kurstaki HD1 strain produces Cry toxins independently of sporulation.**

As the previous results were obtained in Bt kurstaki strain HD73, the role of VipR in the expression of Cry toxins independently of sporulation in Bt kurstaki strain HD1 (a strain close to the biopesticide Dipel^{®}) has been studied. A *spo0A* gene-deleted HD1 strain was constructed. In this strain, SigE and SigK are therefore not produced and toxin expression can only occur via VipR activity, if this expression pathway is conserved between the 2 strains. Toxin production in this genetic context has been studied by protein analysis on SDS-PAGE gel. The presence of toxins migrating at the same size as control samples containing either Cry2Aa or Cry1Ac toxin **(****Fig. 5A****)** has been observed. Western blot analysis using antibodies recognizing Cry1A and Vip3A proteins confirmed the production of these toxins by HD1 and HD1 *Δspo0A* strains **(****Fig. 5B****).** Mass spectrometry analysis confirmed that peptides specific for the insecticidal proteins Cry1Aa, Cry1Ab, Cry1Ac, Cry1I, Cry2Aa, Cry2Ab and Vip3Aa were present in a total protein sample from strain HD1 *Δspo0A.* The number of peptides identified and the overlap of these peptides on the majority of the protein sequences corresponding to the protein toxins cited above confirm the effective production of all insecticidal proteins by the sporulation mutant of strain HD1. Microscopic observations showed that HD1 *Δspo0A* produced crystalline inclusions in non-spore-forming cells **(****Fig. 5C****).** Similar to strain HD73 *Δspo0A,* part of the bacterial population of strain HD1 *Δspo0A* lyses during the stationary phase of growth as numerous crystals could be observed in the extracellular medium. This lysis phenotype is linked to the presence of 3 *ami* genes (*ami299* of SEQ ID N°6, *ami65* of SEQ ID N°8 and *omi95* of SEQ ID N°7, the equivalent of *ami73*) controlled by VipR in the genome of this strain. The deletion of these genes, in addition to the *spo0A* mutation, can therefore produce a strain which produces toxin crystals and which does not lyse, trapping the toxins in a cell envelope.

### Presence of VipR-Box and the vipR gene in the genome of the biopesticide strain, Bt aizawai

Bioinformatics analysis concluded that *vipR* and VipR-binding boxes were present in the promoter regions of insecticidal toxin genes in the genome of another strain used as a biopesticide, Bt aizawai ABTS-1857 (Xentari^{®}).

In this strain too, the *vipR, vip3A, cry2Ab, cry1Ia* genes and two *ami* genes located upstream of a *cry1Aa* gene and a *cry1Ab* gene are preceded by a VipR-box **(****Fig. 1A****)** corresponding to the identified consensus (Chen, Verplaetse et al., 2022).

Accordingly, such strain could also be modified to prevent the formation of spores and abolish the expression or activity of Ami proteins.

### SEQUENCE LISTING

Bacillus thuringiensis serovar kurstaki strain HD73 - SEQ. ID. N°3 *- ami73* 
MTVFNTKEINNTPFESNIIPKGNRNRPAYSMNPRYITIHTTANQNAGTDARAHARYVNNGGGSSSVSWHFTVD
DNRIVQHLPLNENGWHAGDGNGAGNRSSIGIEICENRDGNFEKALTHAAGLVKFLMAHLGLKIGNVVSHQH
WSGKNCPRPIFNRPGGFEGFKRMIEDSLNDMVVENHNKDAGTQGLGIAYMEGVNINLRRGPSTSSDVIRKLN
KPESYIVWEEREGWLNLGNSWVKYDSSYIYFARRQTSNIGKLVVVDTNELWVYGSANWNDKIKTVKKGEAFTI
QDELQVNGSNMYKCKYFYITTNSQFVYVK
Bacillus thuringiensis serovar aizawai strain ABTS-1857 - SEQ. ID. N°4 
MTVFNVKEINKIPFESNIIPKGNRNRPAYTMNPRYITIHTTANQNAGADARAHARYVNNGGGSSGVSWHFTVD
DHRIVQHLPLNENGWHAGDGNGAGNRSSIGIEICENRDGNFEKALTHAAGLVKFLMAHLGLKIGNVVSHQHW
SGKNCPRPIFNRPGGFEGFKRMIEDSLNDMVVENHNKDAGTQGLGIAYMEGVNINLRRGPSTSSDVIRKLNKP
ESYIVWEEREGWLNLGNSWVKYDSSYIYFARRQTSNIGKLVVVDTNELWVYGSANWNDKIKTVKKGEAFTIQD
ELQVNGSNMYKCKYFYITANSQFVYVK
Bacillus thuringiensis serovar aizawai strain ABTS-1857 - SEQ. ID. N°5 
MTVFNTKEINNTPFESNIIPKGNRNRPAYSMNPRYITIHTTANQNAGTDARAHARYVNNGGGSSSVSWHFTVD
DNRIVQHLPLNENGWHAGDGNGAGNRSSIGIEICENRDGNFEKALTHAAGLVKFLMAHLGLKIGNVVSHQH
WSGKNCPRPIFNRPGGFEGFKRMIEDSLNDMVVENHNKDAGTQGLGIAYMEGVNINLRRGPSTSSDVIRKLN
KPESYIVWEEREGWLNLGNSWVKYDSSYIYFARRQTSNIGKLVVVDTNELWVYGSANWNDKIKTVKKGEAFTI
QDELQVNGSNMYKCKYFYITTNSQFVYVK
Bacillus thuringiensis serovar kurstaki strain HD1_Amidase_pBMB299 - SEQ. ID. N°6 - ami299 
MTVFNTKEINNTPFESNIIPKGNRNRPAYSMNPRYITIHTTANQNAGADARAHARYVNNGGGSSGVSWHFTV
DDNRIVQHLPLNENGWHAGDGNGAGNRSSIGIEICENRDGNFEKALTHAAGLVKFLMAHLGLKIGNVVSHQH
WSGKNCPRPIFNRPGGFEGFKRMIEDSLNDMVVENHNKDAGTQGLGIAYMEGVNINLRRGPSTSSDVIRKLN
KPESYIVWEEREGWLNLGNSWVKYDSSYIYFARRQTSNIGKLVVVDTNELWVYGSANWNDKIKTVKKGEAFTI
QDELQVNGSNMYKCKYFYITTNSQFVYVK
Bacillus thuringiensis serovar kurstaki strain HD1_Amidase_pBMB95 - SEQ. ID. N°7 - ami95 
MTVFNVKEINKIPFESNIIPKGNRNRPAYTMNPRYITIHTTANQNAGADARAHARYVNNGGGSSGVSWHFTVD
DHRIVQHLPLNENGWHAGDGNGAGNRSSIGIEICENRDGNFEKALTHAAGLVKFLMAHLGLKIGNVVSHQHW
SGKNCPRPIFNRPGGFEGFKRMIEDSLNDMVVENHNKDAGTQGLGIAYMEGVNINLRRGPSTSSDVIRKLNKP
ESYIVWEEREGWLNLGNSWVKYDSSYIYFARRQTSNIGKLVVVDTNELWVYGSANWNDKIKTVKKGEAFTIQD
ELQVNGSNMYKCKYFYITANSQFVYVK
Bacillus thuringiensis serovar kurstaki strain HD1_Amidase_pBMB65 - SEQ. ID. N°8 - ami65 
MTVFNTKEINNTPFESNIIPKGNRNRPAYSMNPRYITIHTTANQNAGTDARAHARYVNNGGGSSSVSWHFTVD
DNRIVQHLPLNENGWHAGDGNGAGNRSSIGIEICENRDGNFEKALTHAAGLVKFLMAHLGLKIGNVVSHQH
 
WSGKNCPRPIFNRPGGFEGFKRMIEDSLNDMVVENHNKDAGTQGLGIAYMEGVNINLRRGPSTSSDVIRKLN
KPESYIVWEEREGWLNLGNSWVKYDSSYIYFARRQTSNIGKLVVVDTNELWVYGSANWNDKIKTVKKGEAFTI
QDELQVNGSNMYKCKYFYITTNSQFVYVK
Bacillus thuringiensis serovar galleriae strain HD-29_Amidase_pBMB126 - SEQ. ID. N°9 
MTVFNVKEINKIPFESNIIPKGNRNRPAYTMNPRYITIHTTANQNAGADARAHARYVNNGGGSSGVSWHFTVD
DHRIVQHLPLNENGWHAGDGNGAGNRSSIGIEICENRDGNFEKALTHAAGLVKFLMAHLGLKIGNVVSHQHW
SGKNCPRPIFNRPGGFEGFKRMIEDSLNDMVVENHNKDAGTQGLGIAYMEGVNINLRRGPSTSSDVIRKLNKP
ESYIVWEEREGWLNLGNSWVKYDSSYIYFARRQTSNIGKLVVVDTNELWVYGSANWNDKIKTVKKGEAFTIQD
ELQVNGSNMYKCKYFYITTNSQFVYVK
*Bacillus thuringiensis* strain Berliner ATCC 10792_Amidase_poh4- SEQ. ID. N°10 
MTVFNTKEINNTPFESNIIPKGNRNRPAYSMNPRYITIHTTANQNAGTDARAHARYVNNGGGSSSVSWHFTVD
DNRIVQHLPLNENGWHAGDGNGAGNRSSIGIEICENRDGNFEKALTHAAGLVKFLMAHLGLKIGNVVSHQH
WSGKNCPRPIFNRPGGFEGFKRMIEDSLNDMVVENHNKDAGTQGLGIAYMEGVNINLRRGPSTSSDVIRKLN
KPESYIVWEEREGWLNLGNSWVKYDSSYIYFARRQTSNIGKLVVVDTNELWVYGSANWNDKIKTVKKGEAFTI
QDELQVNGSNMYKCKYFYITTNSQFVYVK
*Bacillus thuringiensis* serovar alesti strain BGSC 4C1_Amidase_ pBMB267- SEQ. ID. N°11 
MTVFNVKEINNTPFESNIIPKGNRNRPAYSMNPGYITIHTTANQNAGADARAHARYVNNGGGSTGVSWHFTV
DDNRIVQHLPLNENGWHAGDGNGAGNRSSIGIEICENRDGNFEKALTHAAGLVKFLMAHLGLKIGNVVSHQH
WSGKNCPRPIFNRPGGFEGFKRMIEDSPNDMVVENHNKDAGTQGLGIAYMEGVNINLRRGPSTSSDVIRKLN
KPESYIVWEERDGWLDLGNSWVKYDPSYIYFARRKTSNVGRLVVVDANELWVYGSADWNNRVKTVKKGEAFT
ILDELQINGSNMYKCKYFYITANSQFVYVK
*Bacillus thuringiensis* serovar chinensis strain CT43_Amidase_pCT281- SEQ. ID. N°12

MTVFNVKEINKIPFESNIIPKGNRNRPAYTMNPRYITIHTTANQNAGADARAHARYVNNGGGSSGVSWHFTVD
DHRIVQHLPLNENGWHAGDGNGAGNRSSIGIEICENRDGNFEKALTHAAGLVKFLMAHLGLKIGNVVSHQHW
SGKNCPRPIFNRPGGFEGFKRMIEDSLNDMVVENHNKDAGTQGLGIAYMEGVNINLRRGPSTSSDVIRKLNKP
ESYIVWEEREGWLNLGNSWVKYDSSYIYFARRQTSNIGKLVVVDTNELWVYGSANWNDKIKTVKKGEAFTIQD
ELQVNGSNMYKCKYFYITANSQFVYVK

## Claims

1. Genetically modified non-sporulating and non-lysing *Bacillus thuringiensis* strain comprising one or more *ami* genes each coding for an Ami protein (i) having at least 70%, preferably at least 75%, 80%, 85%, 90%, 95%, 98% or 100% identity with the sequence SEQ ID N°1 and (ii) whose transcription is regulated by the VipR regulator,
wherein the expression or activity of said protein is abolished.

2. Genetically modified non-sporulating and non-lysing *Bacillus thuringiensis* strain according to claim 1, wherein said strain is from a serovar selected in the group consisting of *kurstaki, aizawai, thuringiensis, chinensis, galleriae* and *alesti.*

3. Genetically modified non-sporulating and non-lysing *Bacillus thuringiensis* strain according to claim 1 or claim 2, wherein said strain is derived from Bt *aizawai* ABTS-1857, 7.29 or GC91 strain or from Bt *kurstaki* HD1, HD73, SA-11, SA-12, PB54, EG2348 or ABTS-351 strain.

4. Genetically modified non-sporulating and non-lysing *Bacillus thuringiensis* strain according to anyone of the preceding claims, wherein the sporulation gene chosen from *spoOA, sigE, sigF, sigH* and *sigK* is inactivated by interruption or modification of the sequence or by deletion of all or part of the gene or wherein the activity of at least one protein encoded by these genes is abolished.

5. Genetically modified non-sporulating and non-lysing *Bacillus thuringiensis* strain according to anyone of the preceding claims, wherein the regulation of the transcription by the VipR regulator involves a VipR-box corresponding to the sequence: 5' TTC-N-N-N-N-AT-N-G-N-N-GAA-N-N-TAT-N-T-N-T-N-CTTT 3' (SEQ ID N°2), wherein N is A or T or C or G, and
wherein said sequence of SEQ ID N°2 is positioned upstream between 15 and 21 nucleotides upstream of the Sigma A-specific -10 box preceding the start codon of said *ami* gene and more preferably 17 nucleotides.

6. Genetically modified non-sporulating and non-lysing *Bacillus thuringiensis* strain according to anyone of the preceding claims, wherein said Ami proteins are proteins of SEQ. ID. N°3, 4, 5, 6, 7, 8, 9, 10, 11 and 12.

7. Genetically modified non-sporulating and non-lysing *Bacillus thuringiensis* strain according to anyone of the preceding claims, wherein said strain comprises at least one *cryl, cry2,* or *vip3A* genes.

8. Method for preparing a genetically modified non-sporulating and non-lysing *Bacillus thuringiensis* strain according to anyone of claims 1 to 7, comprising the step of mutagenesis such that each *ami* gene encoding a protein having at least 70%, preferably at least 75%, 80%, 85%, 90%, 95%, 98% or 100% identity with the sequence SEQ ID N°1 or its promoter is absent or present in a truncated form for abolishing in said bacterium the expression and/or activity of said proteins.

9. Use of a genetically modified non-sporulating and non-lysing *Bacillus thuringiensis* strain according to anyone of claims 1 to 7, as a biopesticide.

10. The use of a genetically modified non-sporulating and non-lysing *Bacillus thuringiensis* strain according to claim 9, for protecting cultures against lepidopteran and/or coleopteran insects.
